# EUROPEAN PATENT APPLICATION

(11) **EP 1 182 208 A2**
(43) Date of publication of application: **27.02.2002**
(21) Application number: 01117620.3
(22) Date of filing: 24.07.2001
(51) Int. Cl.: C07K 14/00, A61K 47/48, A61K 9/127, C12N 15/87

(54) **Composition for DNA transfection**

(30) Priority: 28.07.2000 US 222095 P; 14.11.2000 US 712526
(71) Applicant: Roche Diagnostics GmbH, 68305 Mannheim (DE); F. HOFFMANN-LA ROCHE AG, 4070 Basel (CH)
(72) Inventor: Schlaeger, Ernst-Juergen, 79588 Effringen-Kirchen (DE); Kitas, Eric Agirios, 4144 Arlesheim (CH)

(57) **Abstract**

A novel transfection reagent is disclosed comprising a conjugate of a lipid and a basic membrane disturbing peptide of the formula 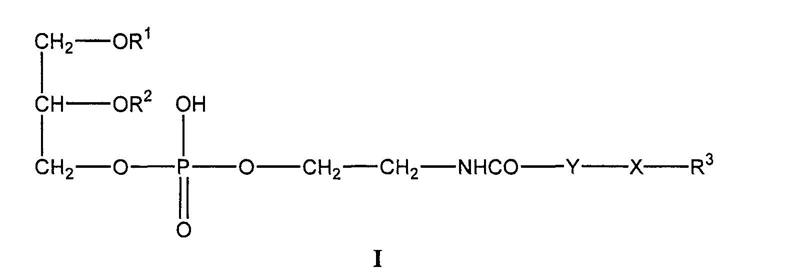 wherein R¹ and R² are selected from the group consisting of hydrocarbyl moieties of straight-chain or branched-chain, saturated or unsaturated aliphatic carboxylic acids and phospholipid moieties, R³ is a basic, membrane-disturbing peptide with a reversed amide backbone, Y is a C₂₋₁₀ alkylene, and X is selected from the group consisting of ―C(O)―NH―, ―S―S―, and salts thereof. Particularly preferred compounds are those wherein R¹ and R² independently are an acyl moiety of a C₁₂₋₂₀ carboxylic acid, especially wherein R¹ and R² are oleoyl. Further disclosed is a transfection method in which transfection efficiency and DNA complex formation are enhanced by the use of a compound of the invention in conjunction with an amino acid concentrate in the form of a protein hydrolysate.

## Description

### BACKGROUND OF THE INVENTION

This invention relates to novel compounds and reagent compositions useful for non-viral introduction of biologically active molecules such as DNA, RNA, peptides and proteins into eukaryotic cells, and more specifically, to compounds and compositions useful in lipid transfection.

Gene transfer technology has become a field of considerable interest. Introduction of an exogenous gene into a cell, i.e., transfection, bears many important scientific and medical applications, from gene regulation and the production of recombinant proteins to gene therapy.

Viruses have evolved to bypass the different cellular barriers to gene transfer and have indeed become vectors of choice for transfection. Many viruses, including retrovirus, adenovirus and herpes virus, are now engineered to carry therapeutic genes and are used in human clinical trials for gene therapy. However, there remains a risk of infectious and immunologic reaction, and the large scale production of viruses is difficult and time consuming.

For these various reasons, non-viral systems have been developed to carry DNA into cells, e.g., the transfection technique based on a cationic lipid, the dioleoyloxypropyl trimethylammonium (Felgner et al., *Proc. Natl. Acad. Sci. USA 84,* 7413-7417, 1987) commercialized as Lipofectin™. Since the discovery of this transfection technique, many more cationic lipids have been synthesized, and some are commercially available as transfecting reagents for laboratory use. Some examples are DOGS (Transfectam™), DOSPA (Lipofectamine™) and DOTAP (DOTAP™).

Nevertheless, despite important progress in the formulation of non-viral gene delivery systems, there remains a need for more efficient techniques since the transfection efficiency of synthetic systems is usually below that of viral vectors. Furthermore, many problems arise *in vivo,* and the poor stability of the non-viral systems in biological fluids does not allow high and reproducible levels of transfection *in vivo.*

Transfection of cells with oligonucleotides such as DNA can be used, for example, to express in a host cell or organism a protein which is not normally expressed by that cell or organism. For example, a DNA molecule called a plasmid may be introduced into a cell that does not normally contain the gene(s) encoded by that plasmid in order to express a marker gene product in that cell, or to express a protein of interest such as a recombinant protein which is later harvested from such cells (Sambrook et al., Molecular Cloning: A Laboratory Manual, 2^{nd} ed., Cold Spring Harbor, 1989). The transfection of oligonucleotides into cells can also be used therapeutically. For example, antisense oligonucleotides, once in the cell or cell nucleus, bind to target single-stranded nucleic acid molecules by Watson-Crick base pairing or to double stranded nucleic acids by Hoogsteen base pairing, and in so doing, disrupt the function of the target by (a) preventing the binding of factors required for normal transcription, splicing or translation, (b) triggering the enzymatic degradation of mRNA by RNAse H, or (c) destroying the target via reactive groups attached directly to the antisense oligonucleotide (Zamecnic et al., *Proc. Natl. Acad. Sci. USA 75,* 280-284, 1978). Gene therapy or DNA-based vaccination are other therapeutic applications.

Proteins and other macromolecules are transfected into cells for therapeutic and screening purposes. For example, immunization is enhanced by introducing an immunogenic protein into a cell so that it is more efficiently processed and presented on the surface of the cell, thereby enhancing the immunogenic response. Negatively charged macromolecules which act inside a cell are transported past the cell membrane into the cytoplasm where they exert their effect. Factors which enhance or hinder transcription of DNA can be used in a screening test to verify the transcription of a gene of interest. These transcription assays are well known for use in screening compounds to determine their effect against a particular macromolecule, for example, a cell receptor.

Cell-lytic antibacterial peptides that act by perturbing the barrier function of membranes are reviewed in Saberwal et al., *Biochim. Biophys. Acta 1197*, 109-131, 1994. Certain fatty acid-bearing basic peptides having antibacterial activity are disclosed in Vogler et al., *Helv. Chim. Acta 47*, 526-543, 1964. Poly(lysine-serine) random polymers for use as carriers to transport oligonucleotides into cells are disclosed in European Patent Publication No. EP-A-727 223.

European Patent Publication No. EP-A-784 984 and Legendre et al., *Bioconjugate Chem. 8*, 57-63, 1997, describe conjugates of a lipid and a basic, membrane-disturbing peptide that bind polynucleotides and anionic macromolecules and that can be used for transfection of cells. The peptide portion of the conjugate consists of natural amino acids linked by a natural amide binding.

Nevertheless, despite important progress in the formulation of non-viral gene delivery systems, there remains a need for more efficient techniques, since the transfection efficiency of synthetic systems is usually below that of viral vectors. Furthermore, many problems arise *in vivo* and *in vitro* due in part to the poor stability of the non-viral systems in biological fluids, and culture media does not allow high and reproducible levels of transfection.

Many known compounds used for transfection experiments are toxic for eukaryotic cells. In addition problems may occur in scaling up where the conditioned medium reduces the effect of known transfecting agents.

The present invention is directed to novel compositions which avoid disadvantages associated with known transfection agents.

In one aspect, the invention is concerned with novel compounds which are conjugates of lipids and a modified basic membrane-disturbing peptide, characterized in that the peptides comprise a reversed amide backbone.

The term "conjugate" means a compound consisting of a lipid chemically bound to a peptide, e.g., via a disulfide bond formed between a sulfur atom present in or attached to the lipid and a sulfur atom present in or attached to the peptide, or an amide bond formed between a carboxyl group present in or attached to the lipid and an amino group of the peptide.

The term "lipid" as used herein comprises straight chain, branched-chain, saturated or unsaturated aliphatic carboxylic acids and phospholipids. Examples of aliphatic carboxylic acids are lauric acid, palmitic acid, stearic acid, oleic acid and (CH₃(CH₂)ₙ)₂CHCOOH, where n is an integer from 3 to 19. Examples of phospholipids are phosphatidylethanolamines such as dioleoylphosphatidylethanolamine.

The term "basic peptide" denotes a peptide containing at least one basic amino acid. Examples of such basic amino acids are natural and non-natural diaminomonocarboxylic acids such as alpha- and beta-diaminopropionic acid, alpha- and gamma-diaminobutyric acid, lysine, arginine, ornithine and p-aminophenylalanine.

The term "membrane-disturbing peptide" denotes a cell-lytic or antibacterial peptide that perturbs the barrier function of membranes (G. Saberwal and R. Nagaraj, *BBA 1197*, 109-131, 1994). Examples of basic, cell-lytic peptides are melittin, hemolysin, mastoparan, bombolitin, crabrolin and derivatives thereof. Examples of basic antibacterial peptides are cecropins, magainins, gramicidin S, tyrocidine and derivatives thereof.

The term "derivative" refers to a peptide wherein one or more amino acid residues are either missing, have been added or have been replaced by another amino acid residue without substantially changing the biological activity of the original peptide concerned, i.e., it allows transfection of a macromolecule, preferably a polynucleotide, into a cell. The term "derivative" also refers to a peptide wherein the terminal carboxyl group is esterified, particularly to form lower alkyl esters such as the methyl and ethyl ester, or converted into an amide, lower alkyl amide or di-lower alkyl amide or hydrazide. The term "derivative" also relates to a peptide wherein the NH₂ group of the N-terminus may be acylated to form an amide or a lower alkyl amide. In particular, the NH₂ group is acetylated. The term "lower" denotes groups containing from 1-6 carbon atoms.

The term "reversed amide backbone" refers to a retro-peptide which characteristically has the same composition as its parent peptide, but the sequence is reversed, i.e., n, ... 3, 2, 1 instead of 1, 2, 3, ... n when reading in the N to C direction. Both have normal peptide bonds.

Throughout this application, the following standard abbreviations are used to refer to amino acids:

| | | | |
|---|---|---|---|
| Alanine | Ala | D-Alanine | D-Ala |
| Glutamate | Glu | D-Glutamate | D-Glu |
| Glutamine | Gln | D-Glutamine | D-Gln |
| Aspartate | Asp | D-Aspartate | D-Asp |
| Asparagine | Asn | D-Asparagine | D-Asn |
| Leucine | Leu | D-Leucine | D-Leu |
| Glycine | Gly | | |
| Lysine | Lys | D-Lysine | D-Lys |
| Serine | Ser | D-Serine | D-Ser |
| Valine | Val | D-Valine | D-Val |
| Arginine | Arg | D-Arginine | D-Arg |
| Threonine | Thr | D-Threonine | D-Thr |
| Proline | Pro | D-Proline | D-Pro |
| Isoleucine | Ile | D-Isoleucine | D-Ile |
| Methionine | Met | D-Methionine | D-Met |
| Phenylalanine | Phe | D-Phenylalanine | D-Phe |
| Tyrosine | Tyr | D-Tyrosine | D-Tyr |
| Cysteine | Cys | D-Cysteine | D-Cys |
| Tryptophan | Trp | D-Tryptophan | D-Trp |
| Histidine | His | D-Histidine | D-His |
| Ornithine | Orn | D-Ornithine | D-Orn |

### SUMMARY OF THE INVENTION

In a preferred embodiment, the novel compounds of the present invention are compounds of the formula 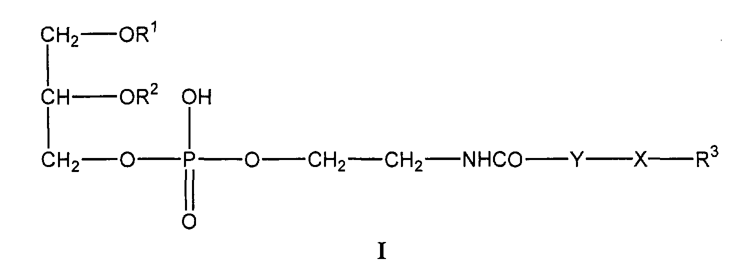 wherein R¹ and R² are selected from the group consisting of hydrocarbyl moieties of straight-chain and branched-chain, saturated and unsaturated aliphatic carboxylic acids and phospholipid moieties, R³ is a basic, membrane-disturbing peptide with a reversed amide backbone, Y is a C₂₋₁₀ alkylene, and X is selected from the group consisting of -C(O)-NH-, -S-S-, and salts thereof.

Particularly preferred compounds are those wherein R¹ and R² independently are an acyl moiety of a C₁₂₋₂₀ carboxylic acid. The term "C₁₂₋₂₀" denotes a number of carbon atoms of from 12 to 20. The acyl moieties R¹ and R² can be straight chain or branched-chain, saturated or unsaturated moieties. Examples of such moieties are lauroyl, palmitoyl, stearoyl and oleoyl. In a preferred aspect, R¹ and R² are oleoyl. Y is preferably ethylene, propylene or decamethylene. X is preferably -S-S-.

R³ is a basic, membrane-disturbing peptide with a reversed amide backbone. For example, R³ is Gln-Gln-Arg-Lys-Arg-Lys-Ile-Trp-Ser-Ile-Leu-Ala-Pro-Leu-Gly-Thr-Thr-Leu-Val-Lys-Leu-Val-Ala-Gly-Ile-NH-CH[CONH₂]-(CH₂)-. The peptide has a reversed amide backbone. Preferably, R³ comprises at least 50%, more preferably 65%, and even more preferably 80% D-amino acids or derivatives thereof. In the most preferred embodiment, all amino acids are D-amino acids. The term "D-amino acid" refers to naturally as well as non-naturally occurring D-α-amino carbonic acids and derivatives thereof. In addition, R³ also comprises membrane-disturbing peptide sequences such as magainin, cecropin, defensins, etc., or chimers of such, e.g., cecropin-melittin (Hancock et al., *TIBTECH 16,* 82-88, 1998) synthesized as the retro-inverso peptides and derivatized to allow conjugation to lipids, analogous to the methods as described below, especially analogous to Examples 1 and 2.

The term "retro-inverso peptide" means the all -D analog of an all -L retro-peptide.

In a further preferred embodiment, R³ is D-Gln-D-Gln-D-Arg-D-Lys-D-Arg-D-Lys-D-Ile-D-Trp-D-Ser-D-Ile-D-Leu-D-Ala-D-Pro-D-Leu-D-Gly-D-Thr-D-Thr-D-Leu-D-Val-D-Lys-D-Leu-D-Val-D-Ala-D-Gly-D-Ile-NH-CH[CONH₂]-CH-(CH₂)-.

The most preferred compound is

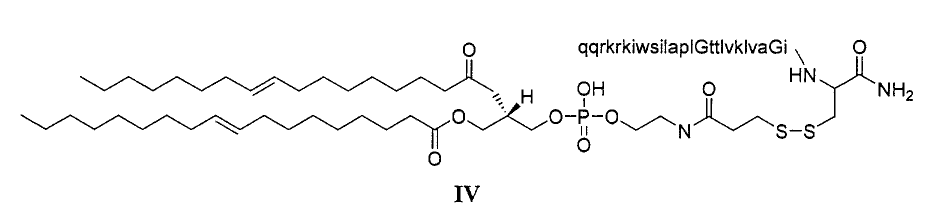

A further embodiment of the present invention refers to the peptide portion of R³, especially to the intermediate peptide Gln-Gln-Arg-Lys-Arg-Lys-Ile-Trp-Ser-Ile-Leu-Ala-Pro-Leu-Gly-Thr-Thr-Leu-Val-Lys-Leu-Val-Ala-Gly-Ile-Cys-NH₂ and derivatives, salts and solvates thereof having a reversed amide backbone and consisting of at least 50%, more preferably 65%, and even more preferably 80% of D-amino acids or derivatives thereof. In a most preferred embodiment, all amino acids are D-amino acids. The term "D-amino acid" refers to naturally as well as non-naturally occurring D-α-amino carbonic acids and derivatives thereof.

In a preferred embodiment, the peptide is D-Gln-D-Gln-D-Arg-D-Lys-D-Arg-D-Lys-D-Ile-D-Trp-D-Ser-D-Ile-D-Leu-D-Ala-D-Pro-D-Leu-D-Gly-D-Thr-D-Thr-D-Leu-D-Val-D-Lys-D-Leu-D-Val-D-Ala-D-Gly-D-Ile-D-Cys-NH₂.

In another aspect, this invention relates to a process for preparing the novel compounds defined above, i.e., conjugates of lipids and basic, membrane-disturbing peptides wherein the peptides comprise a reversed amide backbone, and compositions comprising at least one such compound, a polynucleotide or any other anionic macromolecule, and, optionally, a helper lipid, a short chain phospholipid, or a cationic lipid and optionally an additional known transfection reagent other than a conjugate of this invention, i.e., a compound of formula I or II. In still another aspect, this invention relates to compositions comprising conjugates of lipids and basic, membrane-disturbing peptides and a helper lipid, a short chain phospholipid, or a cationic lipid and optionally an additional known transfection reagent other than a conjugate of this invention, e.g., a compound of formula I. The invention further relates to the use of the novel compounds as a carrier for transfecting a cell with a polynucleotide or any other anionic macromolecule. Even further, the invention relates to the enhancement of transfection efficiency when DNA complex formation is performed in the presence of additional amino acids, preferably in the form of a protein hydrolysate.

### DETAILED DESCRIPTION OF THE INVENTION

The compounds provided by this invention can be prepared by reacting a peptide of the formula R³NH₂ with a lipid of the formula 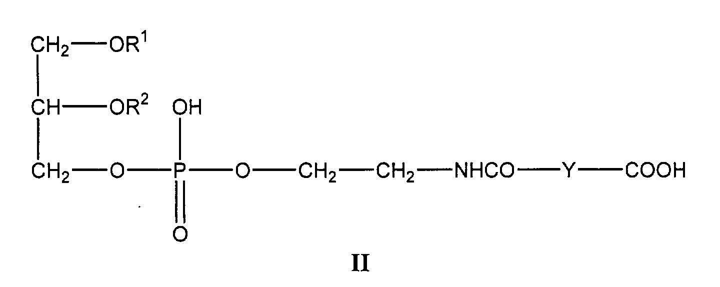 or a peptide of the formula R³SH with a lipid of the formula 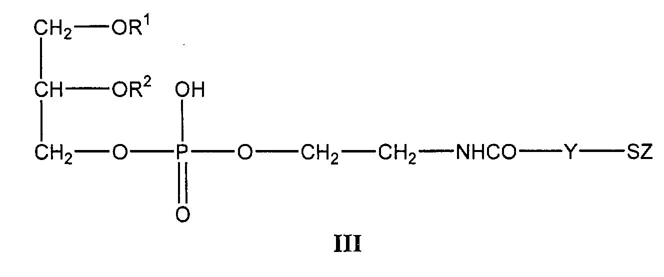 wherein R¹, R², R³ and Y are as defined above and Z is a leaving group such as 2-pyridinethio. These reactions can be carried out in a manner known per se.

Compounds of the formula R³NH₂ wherein the peptide part comprises a reversed amide backbone may be prepared by methods known in the art. The corresponding methods have been described, for example, for the preparation of synthetic melittin, its enantio-, retro- and retroenantio-isomers and derivatives thereof (Juvvadi et al., *J. Am. Chem. Soc. 118*, 8989-8997, 1996).

The peptides may be prepared by the solid-phase synthesis technique. In this technique, synthesis occurs while the peptide is attached to a polymeric support, thereby allowing for the separation of product from byproduct by washing steps. The completion of the acylation reaction is ensured by using large excesses of soluble reagents. Synthesis involves the covalent anchorage of the first amino acid in the sequence to the solid support followed by the deprotection of the protected amino function for subsequent coupling to the incoming amino acid derivative. After *n* cycles of deprotection and coupling, the peptide is released from the solid phase by a chemical cleavage reaction. In a preferred embodiment, D-amino acids are used.

Thus, the coupling of a peptide of the formula R³NH₂ with a lipid of the formula II can be accomplished by reacting the compounds wherein an amino group other than the amino group to be reacted are protected in a suitable solvent in the presence of a condensation agent such as dicyclohexylcarbodiimide analogous to known methods for producing peptide bonds.

The reaction of a peptide of the formula R³SH with a compound of the formula R-SZ can be carried out in an appropriate solvent or solvent mixture which solubilizes both reactants. The compound of the formula R-SZ can be dissolved in an organic solvent, e.g., in chloroform. The peptide R³SH is suitably dissolved in an aqueous buffer solution, such as phosphate buffer, that contains an appropriate amount of a water-miscible organic solvent such as acetonitrile to accomplish the formation of a single phase reaction system.

Compounds of the formula II and III are known or can be prepared by known methods, e.g. as described in *Biochim. Biophys. Acta 862,* 435-439, 1986. For example, compounds of the formula 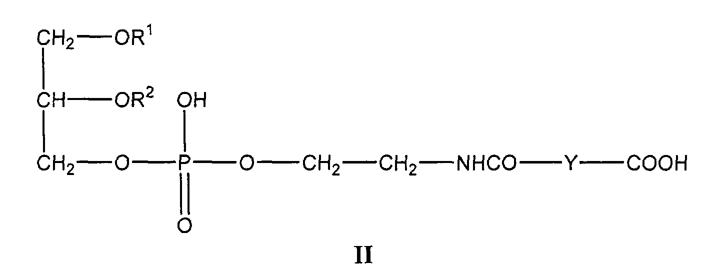 wherein R¹ and R² are oleolyl, and Y is ethylene, propylene or decamethylene and compounds of the formula 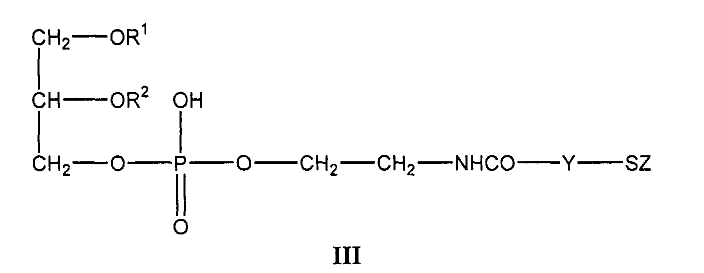 wherein R¹ and R² are oleolyl, Y is ethylene and Z is 2-pyridinethio are commercially available as N-Succinyl-PE, N-Glutaryl-PE, N-Dodecanyl-PE and N-PDP-PE from Avanti Polar Lipids, Alabaster, Alabama.

Any anionic macromolecule can be transfected into a cell using a compound of formula I. An "anionic macromolecule" is a macromolecule which contains at least one negative charge per molecule. Examples of anionic macromolecules which can be transfected in accordance with this invention include polynucleotides, such as deoxyribonucleic acids (DNA) and ribonucleic acids (RNA), and proteins, such as ribonucleoproteins and proteins used for immunization, e.g., viral proteins. Examples of DNA for use in the present invention are plasmids and genes, especially those for which gene therapy protocols have been launched, such as cystic fibrosis transmembrane regulator (CFTR), adenosine deaminase (ADA), thymidine kinase (TK) and HLA B7, as well as reporter genes such as β-galactosidase, luciferase, green fluorescence protein (GFP), chloramphenicol transferase and alpha- antitrypsin. Other examples of DNA are oligodeoxynucleotides and their analogs used as antisense, aptamer or 'triple-helix' agents. Examples of RNA are ribozymes and oligoribonucleotide antisense molecules.

The nature of the cell that is to be transfected is not narrowly crucial. The cell can be a prokaryotic or eukaryotic cell, and a mammalian or a plant cell.

In transfecting a cell using a conjugate of this invention, e.g., a compound of formula I, the cell is contacted with the anionic macromolecule in the presence of an appropriate amount of such compound. The appropriate amount of the conjugate, e.g., a compound of formula I, for a given amount of anionic macromolecule depends on their respective charges. The +/- charge ratio between the conjugate and the molecule to be transfected generally varies between 0.1-10, preferably between 0.5-5. The value of "+/-charge ratio" is calculated by dividing the number of positively charged groups on the amino acids in the group R³ by the number of negative charges of the molecule to be transfected. When the molecule to be transfected is a polynucleotide, for example, the number of negative charges means the number of negatively charged phosphates in the backbone. The optimal ratio within these ranges depends on the cell to be transfected and is readily ascertained by one of skill in the art to which this invention pertains.

The amount of anionic macromolecules to the number of cells is such that the amount of anionic macromolecule for transfecting 10⁴ cells is from 0.1 µg to 10 µg, preferably from 0.2 µg to 2 µg. When the anionic macromolecule is DNA, the preferred amount of DNA for transfecting 10⁴ cells *in vitro* is from 0.1 µg to 10 µg. When cells are being transfected *in vivo,* the preferred amount of DNA is from 0.1 µg to 1 µg.

In a preferred aspect of the invention, the transfection is further carried out in the presence of a helper lipid, short chain phospholipid, cationic lipid or any other known transfection-competent molecule other than a conjugate of this invention. Any conventional helper lipid can be used. "Helper lipids" are phospholipids which are known to increase delivery of macromolecules to cells when used together with known transfection competent molecules. Examples of helper lipids are phospholipids, such as phosphatidylcholines and phosphatidylethanolamines and mixtures thereof. Preferred helper lipids are phosphatidylethanolamines, such as dioleoylphosphatidylethanolamine. Any conventional short chain phospholipid can be used. "Short chain phospholipids" are phospholipids containing fatty acid residues which contain from 6 to 12 carbon atoms in their backbone. Examples of short chain phospholipids are phosphatidylcholines that carry two C₆₋₁₂ fatty acid residues. Preferred short chain phospholipids are dicapryl- and dicapryloyl phosphatidylcholine.

Examples of transfection competent molecules include cationic lipids as described by Behr in *Bioconjugate Chem. 5*, 382-389, 1994 and Gao et al. in *Gene Ther. 2,* 710-722, 1995, polycations as described by Kabanov et al. in *Bioconjugate Chem.* 6, 7-20, 1995, and peptides, polymers and other non-viral gene delivery systems as described by Ledley in *Human Gene Therapy 6*, 1129-1144, 1995.

The helper lipid, short chain phospholipid, cationic lipid and optionally other additional known transfection competent molecule other than a conjugate of this invention is suitably in the form of a liposome, micelle, organic or aqueous dispersion, or an organic or aqueous solution. The optimal molar ratio between the compound of formula I and the helper lipid is 0.1:50, preferably 1:10. The optimal molar ratio between helper lipid and short-chain phospholipid is 2:20. The optimal molar ratio between the compound of formula I or II and additional transfection competent molecules is 0.1:10.

In an especially preferred aspect of the present invention, transfection efficiency is significantly enhanced when DNA complex formation is performed in the presence of a concentrated amino acid mixture in the form of a protein hydrolysate. A preferred protein hydrolysate is a tryptic meat digest, Primatone RL (Sheffield Products, Quest International, Germany). Primatone RL is a medium supplement of a complex nature which serves as a source of amino acids, oligopeptides, iron salts, some lipids and other trace low molecular weight substances. The addition of Primatone RL to mammalian and insect cell culture media is known to improve the cell growth properties of many cell lines. However, it was quite surprising to discover that the addition of Primatone RL to the DNA complex formation medium of the present invention significantly enhanced complex formation. This effect was surprising, in part, because it is well accepted in the art to which the present invention belongs that it is essential that the lipid-nucleic acid complex is formed in the absence of serum, a complex medium supplement containing proteins, peptides, lipids, etc. which are known to interfere with complex formation (*FAQS for Transfections and Cationic Lipid Reagents*, Life Technologies, http://www.lifetech.com). Other preferred protein hydrolysates include Primatone RL-UF (ultrafiltered) and Primatone HS.

The present invention also comprises the use of a composition as defined above for transfecting a eukaryotic or prokaryotic cell *in vivo* or *in vitro* with an anionic macromolecule, preferably with a polynucleotide.

The invention also comprises the use of compounds of formula I as defined above for transfecting a eukaryotic or prokaryotic cell *in vivo* or *in vitro* with an anionic macromolecule, preferably with a polynucleotide.

A further embodiment of the present invention is a process for transfecting a cell *in vivo* or *in vitro* with an anionic macromolecule, preferably a polynucleotide, comprising contacting a cell *in vivo* or *in vitro* with the anionic macromolecule in the presence of a compound of formula I or with a composition as defined above.

In addition, the invention is directed to a process for introducing a biologically active anionic molecule into a cell *in vivo* or *in vitro* with an anionic macromolecule, comprising contacting a cell *in vivo* or *in vitro* with the anionic macromolecule in the presence of a composition or a compound as defined above.

For transfection, an appropriate amount of a conjugate of this invention, e.g., a compound of formula I, is added to the molecule to be transfected, e.g., plasmid DNA, suitably in an aqueous solution. Optionally, a helper lipid and, if desired, a short chain phospholipid, a cationic lipid and optionally another additional known transfection competent molecule other than a conjugate of this invention are then added, either in the form of a liposome, micelle, or as an organic solution or aqueous dispersion. Alternatively, the molecule to be transfected may be added to a composition comprising a compound in accordance with this invention, a helper lipid, and, if desired, a short chain phospholipid, a cationic lipid and optionally another additional known transfection competent molecule other than a conjugate of this invention. The composition may be in solid, liquid, semisolid or aerosol form, suitably in the form of a liposome, micelle, or an organic solution or aqueous dispersion.

For transfecting cells in an animal or human patient, the composition can be administered by oral, parenteral, transdermal, pulmonary, nasal, rectal, ocular, ventricular, vascular and intratumoral routes. Furthermore, the composition can be administered by high velocity impaction administration to the skin surface. The progress of transfection can be measured by appropriate testing protocols which are known to those skilled in the art.

The present invention also relates to a composition comprising at least one compound as defined above and a helper lipid, a short chain phospholipid, a cationic lipid, or optionally another additional transfection competent molecule. In addition, the composition may comprise an anionic macromolecule, preferably a polynucleotide. These compositions may also comprise a polycationic polymer, preferably polyethyleneimine (PEI). The components of these compositions may be in the form of an aqueous or organic solution, an aqueous or organic dispersion, or a liposome or micelle.

The term "polyethyleneimine (PEI)" refers to a synthetic organic, generally branched or linear macromolecule with a high cationic charge/density potential, preferably with a molecular weight of about 25 kDa.

The invention also relates to the use of a compound as above for transfecting a cell with an anionic macromolecule, preferably a polynucleotide.

In accordance with the present invention, it has been found that conjugating a lipid to a basic, membrane-disturbing peptide having a reversed amide bond results in novel compounds that bind polynucleotides and other anionic macromolecules and that can be used for transfection of cells. Thus, the invention is concerned with a process for transfecting a cell with an anionic macromolecule comprising contacting the cell with the anionic macromolecule in the presence of a compound as described above, so as to transfect the cell with the anionic macromolecule.

The invention also relates to a process for production of large quantities of recombinant proteins in PEI-mediated transfected cells. For example, high-level expression of both G protein-coupled receptors and ligand-gated ion channels by the use of Semliki Forest virus (SFV) has been realized. Generally, Bₘₐₓ values of more than 50 pmol receptor per mg protein and receptor densities of more the 3 x 10⁶ receptors per cell have been achieved. To further facilitate large-scale protein production, optimal conditions for mammalian serum-free suspension cultures have been obtained. Adaptation of BHK, CHO and HEK293 cells to these conditions has allowed efficient infection with SFV vectors to produce large volumes of recombinant protein expressing cell cultures.

Surprisingly, the growth temperature of the cell cultures can have a dramatic effect on the duration and levels of recombinant protein expression. Expression of recombinant luciferase is increased 5- to 10-fold by lowering the growth temperature of BHK and CHO cells from 37 °C to 33 °C. The expression time is much longer at 33 °C, with high expression 65 hours post-infection.

Moreover, the effect of the temperature on the expression of two 7TM receptors, human neurokinin-1 receptor and rat metabotropic glutamate receptor-2 and the 5-HT₃ ligand-gated ion channel could be shown. A similar effect as observed for luciferase has also been obtained for the receptors. The receptor density is much higher in cells grown at 33 °C compared to 37 °C. The expression time for receptors is usually restricted to 24 hours in SFV-infected cells, but can be prolonged to 65 hours when cells are cultured at 33 °C. This improvement can greatly facilitate production of large quantities of recombinant proteins. Moreover, the utility of the inventive transfection for the rapid expression of heterologous proteins in large scale for adherent cells (CFU) and suspension cells (12 liter, 23 liter, 60 liter fermentors) has been realized. In particular, helper lipids in combination with PEI exhibit a high transient gene expression in HEK293 cells and other mammalian cell lines at low DNA concentration. In addition, the frequently occurring inhibitory effect of conditioned medium, which is a serious problem for scale up expression methods, is significantly reduced.

The general procedure for protein expression comprises (a) adaptation and growth of a cell line to suspension culture in an appropriate medium and expression vectors, (b) washing of cells to remove heparin/dextran sulfate in preparation for transfection, (c) counting of cells and resuspension in a culture medium appropriate for transfection, (d) preparation of the transfection reagent:DNA complex, maturation of the complex and addition to the cells, and (e) incubation and monitoring of protein expression over 2 to 10 days.

The following examples, which are not meant to be limiting, illustrate the invention further.

### Example 1. Preparation of the retro-inverso peptide R³SH

Continuous-flow solid-phase synthesis was performed on a Pioneer™ Peptide Synthesis System, starting from Tenta Gel S RAM resin (0.25 mmol/g [Rapp Polymere GmbH, Tübingen, Germany] according to the method described by Atherton and Sheppard, Solid Phase Peptide Synthesis: A Practical Approach (IRL Press, Oxford, 1989). The base-labile Fmoc group was used for α-amino protection. Side chains were protected with the following protection groups: D-Arg(Pbf), D-Cys(Trt), D-Gln(Trt), D-Lys(Boc), D-Ser(But), D-Thr-(But) and D-Trp(Boc). Fmoc-amino acids (2.5 equivalents) were activated with an equivalent amount of TATU (L.A. Carpino, *J Am. Chem. Soc. 115,* 4397-4398, 1993) and DIPEA. Fmoc deprotection was achieved with 20% piperidine in DMF. D-Gln(Trt)-D-Gln(Trt)-D-Arg(Pbf)-D-Lys(Boc)-D-Arg(Pbf)-D-Lys(Boc)-D-Ile-D-Trp(Boc)-D-Ser(But)-D-Ile-D-Leu-D-Ala-D-Pro-D-Leu-Gly-D-Thr(But)-D-Thr(But)-D-Leu-D-Val-D-Lys(Boc)-D-Leu-D-Val-D-Ala-Gly-D-Ile-D-Cys(Trt)-amide Tenta Gel S-resin (2.0 g) was treated with a mixture (100 ml) of 90% TFA, 2% EDT, 5% H₂O, 3% triisopropylsilane for 6 hours. The reaction mixture was concentrated and poured into diethyl ether, and the precipitate was collected by filtration and lyophilized from water. The crude peptide was purified by preparative RP-HPLC. There was obtained homogenous D-Gln-D-Gln-D-Arg-D-Lys-D-Arg-D-Lys-D-Ile-D-Trp-D-Ser-D-Ile-D-Leu-D-Ala-D-Pro-D-Leu-D-Gly-D-Thr-D-Thr-D-Leu-D-Val-D-Lys-D-Leu-D-Val-D-Ala-D-Gly-D-Ile-D-Cys-NH₂·6 TFA.

### Example 2. Preparation of a compound of formula IV

The homogenous peptide obtained in Example 1 (24.2 mg, 7 mmol) was dissolved in a mixture of 2 ml of 100 mM ammonium acetate buffer, pH 6.5, and 2 ml of acetonitrile. To this solution there was added 7.4 mg of 1,2-dioleoyl-sn-glycero-3-phosphoethanolamine-N-[3-(2-pyridyldithio)propionate] in 0.5 ml of chloroform. The mixture was stirred at room temperature for 1 hour, and the organic solvent was removed by evaporation. The remaining solution was washed 3 times with ethyl acetate, and the aqueous phase was lyophilized. There was obtained 28.5 mg of a compound of formula IV wherein R¹ and R² are oleoyl and D-Gln-D-Gln-D-Arg-D-Lys-D-Arg-D-Lys-D-Ile-D-Trp-D-Ser-D-Ile-D-Leu-D-Ala-D-Pro-D-Leu-D-Gly-D-Thr-D-Thr-D-Leu-D-Val-D-Lys-D-Leu-D-Val-D-Ala-D-Gly-D-Ile-NH-CH[CONH₂]-CH-(CH₂)- is R³.
ISP-MS:M = 3722.

### Example 3. Transfection using a compound of formula IV

1 mg of the compound obtained in Example 2 was dissolved in 500 µl acetonitrile, diluted in 500 µl sterile pure water (1 mg/ml) and stored at 4 °C. 15 µl of a plasmid solution (1 mg/ml) encoding the soluble human tumor necrosis factor receptor p55 gene was transferred into 1.5 ml medium and mixed. Various amounts of the compound obtained in Example 1 was added, mixed and after 10 minutes at room temperature, the mixture was transferred to 15 ml HEK293-EBNA cells in suspension.

Table 1 shows the transfection efficiency of the compound as described in Example 2 and the cell viability in serum-free suspension culture.

**Table 1**

| Transient transfection of HEK293-EBNA cells | | | | | |
|---|---|---|---|---|---|
| Compound (µg/ml) | 1 | 2 | 4 | 6 | 10 |
| Viability (%) | 90 - 95 | 90 - 95 | 90 - 95 | 90 - 95 | 90 - 95 |
| TNFRp55 (ng/ml) | 0.1 | 0.2 | 2.5 | 6.3 | 83 |

The data clearly indicate that compound-mediated transfection can be achieved in the absence of detectable cell toxicity effects in serum-free suspension culture of HEK293-EBNA cells.

### Example 4. Transfection using a compound of formula IV and PEI

1 mg of the compound obtained in Example 2 was dissolved in 500 µl acetonitrile and diluted in 500 µl sterile pure water (1 mg/ml) and stored at 4 °C. 90 mg polyethyleneimine (PEI), with a molecular weight of 25 kDa, were dissolved in sterile pure water (0.9 mg/ml), neutralized with HCl, sterile filtered and stored at room temperature.

3 µl of a plasmid solution (1 mg/ml) encoding the soluble human tumor necrosis factor receptor p55 gene was diluted in 1.5 ml culture medium and mixed. Various amounts of the compound (1 mg/ml) as described in Example 2 were added, mixed, followed by 8.3 µl PEI (0.9 mg/ml). After mixing and incubation for 10 minutes at room temperature, the mixture was transferred into 15 ml of HEK293-EBNA cells in suspension. Cells which were grown in serum-free suspension culture were transferred into fresh medium before adding the complexes. The released receptor protein was measured 72 hours post-transfection in the culture medium and expressed as ng receptor per milliliter culture.

**Table 2**

| Transient transfection of HEK293-EBNA cells together with PEI | | | | | |
|---|---|---|---|---|---|
| Compound + polyethyleneimine (µg/ml) | 0.02 + 0.5 | 0.05 + 0.5 | 0.1 + 0.5 | 0.15 + 0.5 | 0 + 0.5 |
| TNFRp55 (ng/ml) | 490 | 800 | 1120 | 1100 | 180 |

The results clearly indicate the synergistic enhancement of the compound together with PEI. The sequence of adding first the compound followed by PEI to the DNA or vice versa has no detectable influence on the transfection efficiency. No significant changes of the expression levels were observed when the cells were cultured in the presence of 10% serum.

### Example 5. Luciferase as a measure of transfection efficiency in various cell lines

1 mg of the compound obtained in Example 2 was dissolved in 500 µl sterile pure water (1 mg/ml) and stored at 4 °C. 90 mg polyethyleneimine, with a molecular weight of 25 kDa, was dissolved in sterile pure water (0.9 mg/ml), neutralized with HCl, sterile filtered and stored at room temperature.

3 µl of a plasmid solution (1 mg/ml) encoding the luciferase gene was diluted in 1.5 ml culture medium and mixed. 2.25 µl compound (1 mg/ml) as described in Example 2 was added, mixed and followed by 8.3 µl PEI (0.9 mg/ml). After mixing and incubation for 10 minutes at room temperature, the mixture was transferred into 15 ml of various cells. Cells which were grown in serum-free suspension culture were transferred into fresh medium before adding the complexes. The luciferase activity was measured in the soluble cell extract after 24 hours incubation and expressed as relative light unit (RLU) per milligram protein.

Table 3 shows the transfection efficiency of the compound described in Example 2 in combination with PEI by measuring the luciferase activity in serum-free suspension culture.

**Table 3**

| Luciferase activity (RLU/mg protein) | | | |
|---|---|---|---|
| | Compound | PEI | Compound + PEI |
| BHK 21 Cl | 5.2 x 10⁵ | 2.8 x 10⁷ | 1.8 x 10⁹ |
| CHO dhfr- | 1.4 x 10⁶ | 3.5 x 10⁷ | 5.3 x 10⁹ |
| HEK293-EBNA | 2.2 x 10⁶ | 3.2 x 10⁹ | 6.7 x 10¹⁰ |
| HEK293 | 1.8 x 10⁶ | 2.9 x 10⁹ | 6.3 x 10¹⁰ |
| Glioma C6 | 5.8 x 10⁶ | 6.5 x 10⁷ | 3.4 x 10⁹ |

The results clearly indicate the synergistic enhancement of the compound together with PEI in various cell lines.

### Example 6. GFP as a measure of transfection efficiency in various cell lines

1 mg of the compound obtained in Example 2 was dissolved in 50 µl acetonitrile and diluted in 500 µl sterile pure water (1 mg/ml) and stored at 4 °C. 90 mg polyethyleneimine, with a molecular weight of 25 kDa, was dissolved in sterile pure water (0.9 mg/ml), neutralized with HCl, sterile filtered and stored at room temperature.

3 ml of a plasmid solution (1 mg/ml) encoding the green fluorescence protein was diluted in 1.5 ml culture medium and mixed. 2.25 µl compound (1 mg/ml) was added, mixed and followed by 8.3 µl PEI (0.9 mg/ml). After mixing and incubation for 10 minutes at room temperature, the mixture was transferred into 15 ml of various cells. Cells which were grown in serum-free suspension culture were transferred into fresh medium before adding the complexes. The transfection efficiency was measured by counting the number of fluorescent cells after 24 hours incubation and expressed as percent transfected cells.

Table 4 shows the transfection efficiency of the compound described in Example 2 in combination with PEI by measuring the green fluorescence within various cell lines in serum-free suspension culture.

**Table 4**

| Amount of fluorescence cells (%) - compound + PEI | |
|---|---|
| BHK 21 Cl | 25 (± 5) |
| CHO dhfr- | 15 (± 5) |
| HEK293-EBNA | 85 (± 5) |

### Example 7. Enhancement of transfection efficiency in various cell lines by a compound of formula IV

1 mg of the compound obtained in Example 2 was dissolved in 500 µl acetonitrile and diluted in 500 µl sterile pure water (1 mg/ml) and stored at 4 °C. 90 mg polyethyleneimine, with a molecular weight of 25 kDa, was dissolved in sterile pure water (0.9 mg/ml), neutralized with HCl, sterile filtered and stored at room temperature.

3 ml of a plasmid solution (1 mg/ml) encoding the luciferase gene was diluted in 1.5 ml culture medium and mixed. 2.25 µl compound (1 mg/ml) was added, mixed, and followed by 8.3 µl PEI (0.9 mg/ml). After mixing and incubation for 10 minutes at room temperature, 0.1 ml of the mixture was transferred into a well containing 1 ml medium of a 12-well plate with various adherent cell lines. Cells which were grown in the presence of 10% serum were incubated during 4 hours post-transfection in serum-free medium. The luciferase activity was measured in the soluble cell extract after 24 hours incubation and expressed as relative light unit (RLU) per milligram protein.

Table 5 shows the transfection efficiency of the compound described in Example 2 in combination with PEI by measuring the luciferase activity in various adherent cell lines.

**Table 5**

| Luciferase activity (RLU/mg protein) | | |
|---|---|---|
| | PEI | Compound + PEI |
| HEK293 | 5.4 x 10⁹ | 5.3 x 10¹⁰ |
| Glioma C6 | 3.1 x 10⁹ | 1.9 x 10¹⁰ |
| IMR32 | 2.2 x 10⁹ | 7.7 x 10⁹ |
| COS 7 | 1.1 | 6.3 x 10¹⁰ |
| HepG2 | <1 x 10⁸ | 8 x 10⁸ |

The results clearly indicate also the synergistic enhancement of the compound together with PEI in various adherent cell lines.

In the examples that follow, the following materials and methods were employed:

### Chemicals and expression vectors

Polyethylenimine (PEI), MW 25, was obtained from Aldrich (Buchs, Switzerland). A 10 mM monomer aqueous stock solution was prepared by mixing 9 mg PEI (100% wt/vol of 25 kDa) with 10 ml water, neutralizing with HCl and sterile filtering (Boussif et al., *Proc. Natl. Acad. Sci. 92*, 7297-7301, 1995). The stock solutions were stored at room temperature or at 4 °C for several months.1 mg of the synthetic retro-inverso dioleylmelittin from Example 2 was dissolved in 500 µl acetonitrile (remained turbid) and diluted in 500 µl sterile pure water (became almost clear). The stock reagent was overlaid with nitrogen and stored for several months at 4 °C.

Firefly luciferase and secreted human placental alkaline phosphatase (SEAP) were used as reporter genes. The pGL3-CMV plasmid (Promega) encoding the luciferase was constructed as described recently (Legendre et al., 1996) and the pCMV/SEAP plasmid was obtained from Tropix (USA). Plasmid preparations (*E. coli* DH5 in Terrific Broth) were done using a commercially available miniprep kit (BIO-Rad Laboratories, CA. USA,) and a maxiprep kit (Nucleobond AX, Macherry-Nagel AG, Switzerland). The plasmid concentrations were determined spectrophotometrically and were estimated by (1%) agarose gel electrophoresis calibrated with pUC18 DNA (Pharmacia Biotech, Zürich, Switzerland).

### Cell culture

Human embryonic kidney cells HEK293 (ATCC CRL 1573) and subclone 293-EBNA (Invitrogen) were adapted to serum-free growth in a fortified HL medium (Schumpp et al, *J. Cell Science 97,* 639-647, 1990). The calcium-free base HL medium is a mixture of enforced DHI and RPMI 1640 medium (2:1 by weight) as described recently (Schlaeger, *J. Immunol. Methods 194,* 191-199, 1996) and contains glutamine (5 mM), glucose (3 g/l), insulin (5 mg/l, Sigma Chemie), human transferrin (15 mg/l) (Roche Diagnostics, Rotkreuz, Switzerland), Primatone RL (0.3% v/v from stock solution 10%, Sheffield Products/Quest International, The Netherlands), Pluronic F68 (0.01%, from stock solution 10%) and heparin (30 U/ml, Sigma Chemie). The powder media, calcium-free DHI and calcium-free RPMI 1640, were purchased from Life Technologies (Basel, Switzerland). The feed solution was prepared as described recently (Schlaeger, 1996).

The cells were routinely cultured in spinner flasks at 105 rpm using 70-80% of the recommended working volume (Bellco, Inotech AG, Dottikon, Switzerland). A Variomag stirrer 4B was used in the CO₂ incubator with the Biomodul 40B (Munich, Germany).

### Transfection procedure

HEK293-EBNA cells were routinely grown in the presence of heparin to avoid cell aggregation. For transfection experiments, cells were cultured to a density of 8-12 x 10⁵ cells/ml, centrifuged for 2 minutes at 1100 rpm (Labofuge 400, Heraeus AG, Germany), washed once with heparin-free HL medium (room temperature) and resuspended in heparin- and antibiotic-free medium. The cell concentration was adjusted to 5.5-6 x 10⁵ cells/ml, and the culture was incubated in spinner flasks for at least 4 hours before transfection took place. After addition of the transfection complexes, the cells were incubated for 1-4 days without medium replacement. Two days post-transfection, the cultures were generally fed once daily with a nutrient mixture.

### Preparation of transfection complexes

The DNA complexes were formed in 1/10 of the culture volume in HL medium at room temperature. Under optimized gene delivery conditions for 1 ml cells, 0.2 µg DNA was added to 0.1 ml fresh medium (15 ml Falcon tube) and mixed gently. After two minutes, 0.15 µg of the compound from Example 2 was added and mixed, and after additional two minutes, 0.5 µg PEI was added and mixed. After 15-30 minutes incubation at room temperature, the 0.1 ml transfection complexes were transferred to 1 ml prepared cells, gently shaken and cultured at 37 °C.

Standard transfection experiments were performed using 15 ml cells in a 25 ml spinner flask. 3 µg DNA were added 1.5 ml to HL medium followed by 2.25 µg of the compound from Example 2 and 7.5 µg PEI. After mixing and incubation, the DNA complexes were added to the 15 ml cells and cultured at 37 °C in a CO₂ incubator.

### Product analysis

The luciferase activity was measured generally 20-24 hours post transfection. At each time point, 2 x 1 ml samples were taken and centrifuged for 5 minutes at 2000 rpm at 4 °C, the supernatants decanted and the remaining liquid carefully removed. The cell pellets were resuspended mechanically, and 1 ml ice-cold lysis buffer (Boehringer Mannheim) was added. After mixing, the samples were left on ice for 13 minutes and centrifuged at 12000 rpm for 10 minutes to remove cell debris. The supernatants were frozen at -20 °C until measurement. The samples were thawed, diluted in lysis buffer and analyzed (tube format) using the Lumat LB9501 (Berthold AG, Regensdorf, Switzerland) as described (Legendre et al., 1996).

The protein content of the soluble cell extracts was analyzed using BCA protein assay reagent (Pierce, Rockford, IL, USA).

The SEAP expression was determined in the cell culture supernatant using the chemiluminescent SEAP reporter gene assay (microtiter plate format, Top Count 1 NXT, Packard) according to the protocol of the supplier (Roche Diagnostics, Rotkreuz, Switzerland).

### Example 8. Effect of protein hydrolysate on luciferase expression

1 mg of the compound from Example 2 was dissolved in 0.5 ml acetonitrile mixed for 3-5 minutes at 37 °C, diluted in 0.5 ml sterile, pure water (1 mg/ml) and stored at 4 °C. 90 mg polyethelenimine (PEI), with a molecular weight of 25 kDa, were dissolved in sterile, pure water (0.9 mg/ml), neutralized with HCl and sterile filtered and stored at 4 °C. Primatone RL (sterilized 10% stock solution in pure water) was added to 1.5 ml HL medium (without Primatone) and mixed. After 2-3 minutes, 3 µl of a plasmid solution (1 mg/ml) encoding the luciferase gene was added to the 1.5 ml HL medium and mixed. After 2 minutes incubation at room temperature, 2.25 µl of the compound from Example 2 (1mg/ml) was added and mixed, followed by 8.3 µl PEI (0.9 mg/ml). After mixing and incubation for 15-30 minutes at room temperature, the mixture was transferred into 15 ml of HEK293-EBNA cells. Cells which were grown in serum-free suspension culture in HL medium (containing 0.3% Primatone RL) were transferred into fresh HL medium (containing 0.3% Primatone RL), then adjusted to 5.5-6 x 10⁶ cells/ml before adding the DNA complexes.

The luciferase activity was measured in the soluble cell extract after 22 hrs incubation time and expressed as relative light units per ml culture.

Table 6 shows the increase of the expression levels with increasing amounts of protein hydrolysate in the DNA complex medium (HL) using the compound from Example 2 and PEI.

**Table 6**

| Increased protein hydrolysate concentrations in DNA complex medium (HL) | | |
|---|---|---|
| Protein hydrolysate* (percent) | Luciferase activity (RLU x 10⁹/ml) | Relative increase |
| 0 | 5.72 | 1 |
| 0.05 | 5.81 | 1.01 |
| 0.075 | 7.0 | 1.22 |
| 0.1 | 10.90 | 1.90 |
| 0.15 | 9.91 | 1.73 |
| 0.2 | 6.65 | 1.16 |
| 0.3 | 5.60 | 0.98 |
| 0.45 | 1.81 | 0.31 |

| | | |
|---|---|---|
| * Primatone RL | | |

The results indicated an increase of the luciferase activity in the presence of protein hydrolysate with an optimum of 0.1 % Primatone RL in the DNA complex HL medium.

### Example 9. Effect of protein hydrolysate on luciferase expression in various media

1 mg of the compound from Example 2 was dissolved in 0.5 ml acetonitrile mixed for 3-5 minutes at 37 °C and diluted in 0.5 ml sterile, pure water (1 mg/ml) and stored at 4 °C. 90 mg polyethelenimine (PEI), with a molecular weight of 25 kDa, were dissolved in sterile, pure water (0.9 mg/ml), neutralized with HCl, sterile filtered and stored at 4 °C. Primatone RL (sterilized 10% stock solution in pure water) was added to different 1.5 ml medium (without Primatone) and mixed. After 2-3 minutes, 3 µl of a plasmid solution (1 mg/ml) encoding the luciferase gene was added to the 1.5 ml medium and mixed. After 2 minutes incubation at room temperature, 2.25 µl of the compound from Example 2 (1mg/ml) was added, mixed, followed by 8.3 µl PEI (0.9 mg/ml). After mixing and incubation for 15-30 minutes at room temperature, the mixture was transferred into 15 ml of HEK293-EBNA cells. Cells which were grown in serum-free suspension culture in HL medium (containing 0.3% Primatone RL) were transferred into fresh HL medium (containing 0.3% Primatone RL) then adjusted to 5.5-6 x 10⁶ cells/ml before adding the DNA complexes. The luciferase activity was measured in the soluble cell extract after 20-22 hours incubation time and expressed as relative light units per ml culture.

Table 7 shows the luciferase activities in the presence and absence of protein hydrolysate in different DNA complex media using the compound from Example 2 and PEI.

**Table 7**

| DNA complexes in different media | | | |
|---|---|---|---|
| Medium | Supplier | Luciferase activity (RLU x 10⁹/ml) | |
| | | Without protein hydrolysate* | With protein hydrolysate (0.2%)* |
| HL | Roche | - | 12 |
| DHI | Roche | 6.5 | 10.5 |
| DMEM | Roche/LTI** | 6.4 | 6.4 |
| Dulbecco's Medium w/o phenol red | LTI | 6.42 | 6.5 |
| DMEM/HAM F12 | LTI | 0.025 | 12 |
| IMDM (ISCOVE) | Roche/LTI** | 0.027 | 9.02 |
| MEM | LTI | 3.2 | 4.1 |
| MEM α | LTI | 2.32 | 5.8 |
| OPTI-MEM | LTI | 2.14 | 7.8 |
| RPMI 1640 | LTI | 4.2 | 6.7 |
| IS 293 | Irvine Scientific | 1.84 | 12 |

| | | | |
|---|---|---|---|
| * Primatone RL | | | |
| ** LTI powder, Roche preparation | | | |

The results clearly indicated highly significant variations in the expression levels in different complex media. HL and DHI medium showed the highest activity and relatively low protein hydrolysate stimulation whereas DNA complexes formed in DMEM/HAM F12 medium more then 100-fold increase in the presence of Primatone RL was achieved.

### Example 10. Effect of protein hydrolysate concentration on expression

1 mg of the compound from Example 2 was dissolved in 0.5 ml acetonitrile mixed for 3-5 minutes at 37°C, diluted in 0.5 ml sterile, pure water (1 mg/ml) and stored at 4 °C. 90 mg polyethelenimine (PEI), with a molecular weight of 25 kDa, were dissolved in sterile, pure water (0.9 mg/ml), neutralized with HCl, sterile filtered and stored at 4 °C. Primatone RL (sterilized 10% stock solution in pure water) was added to 1.5 ml DMEM/HAM F12 medium (without Primatone) and mixed. After 2-3 minutes, 3 µl of a plasmid solution (1 mg/ml) encoding the luciferase gene was further added and mixed. After 2 minutes incubation at room temperature, 2.25 µl of the compound from Example 2 (1mg/ml) was added, mixed, followed by 8.3 µl PEI (0.9 mg/ml). After mixing and incubation for 15-30 minutes at room temperature, the mixture was transferred into 15 ml of HEK293-EBNA cells. Cells which were grown in serum-free suspension culture in HL medium (containing 0.3% Primatone RL) were transferred into fresh HL medium (containing 0.3% Primatone RL), then adjusted to 5.5-6 x 10⁶ cells/ml before adding the DNA complexes. The luciferase activity was measured in the soluble cell extract after 20-22 hours incubation time and expressed as relative light units per ml culture.

Table 8 shows the increase of the expression levels with increasing amounts of protein hydrolysate in DMEM/HAM F12 DNA complex medium using the compound from Example 2 and PEI.

**Table 8**

| Increased protein hydrolysate concentrations in DNA complex medium (DMEM/HAM F12) | | |
|---|---|---|
| Protein hydrolysate* (percent) | Luciferase activity (RLU x 10⁹/ml) | Relative increase |
| 0 | 0.029 | 1 |
| 0.1 | 9.47 | 326 |
| 0.2 | 11.6 | 400 |
| 0.3 | 7.95 | 274 |
| 0.4 | 5.92 | 204 |

| | | |
|---|---|---|
| * Primatone RL | | |

The results indicated a significant increase of the expression levels with an optimum of 0.2% protein hydrolysate in DMEM/HAM F12 medium.

### Example 11. Effect of various compounds on luciferase expression

1 mg of the compound from Example 2 was dissolved in 0.5 ml acetonitrile, mixed for 3-5 minutes at 37 °C, diluted in 0.5 ml sterile, pure water (1 mg/ml) and stored at 4 °C. 90 mg polyethelenimine (PEI), with a molecular weight of 25 kDa, were dissolved in sterile, pure water (0.9 mg/ml), neutralized with HCl, sterile filtered and stored at 4 °C.

Different growth promotion compounds were added to 1.5 ml DMEM/HAM F12 medium (without any compounds) and mixed. After 2-3 minutes, 3 µl of a plasmid solution (1 mg/ml) encoding the luciferase gene was added to the 1.5 ml HL medium and mixed. After 2 minutes incubation at room temperature, 2.25 µl of the compound from Example 2 (1mg/ml) was added and mixed, followed by 8.3 µl PEI (0.9 mg/ml). After mixing and incubation for 15-30 minutes at room temperature, the mixture was transferred into 15 ml of HEK293-EBNA cells. Cells which were grown in serum-free suspension culture in HL medium (containing 0.3% Primatone RL) were transferred into fresh HL medium (containing 0.3% Primatone RL) then adjusted to 5.5-6 x 10⁶ cells/ml before adding the DNA complexes. The luciferase activity was measured in the soluble cell extract after 20-22 hours incubation time and expressed as relative light units per ml culture.

Table 9 shows the influence of different compounds on the DNA complex formation in DMEM/HAM F12 medium using the compound from Example 2 and PEI.

**Table 9**

| DNA complexes in DMEM/HAM F12 medium using different compounds | | | |
|---|---|---|---|
| Compound | Final concentration (dilution) | Luciferase activity (RLU x 10⁹/ml) | % Increase |
| Control (without addition) | | 0.019 | 0 |
| Primatone RL | 0.2% | 8.03 | 100 |
| Primatone RL-UF | 0.2% | 8.02 | 99.8 |
| Primatone HS | 0.2% | 8.04 | 100.2 |
| Lactalbumin | 0.3% | 3.64 | 45 |
| HySoy | 0.3% | 5.94 | 73 |
| Yeast extract | 0.3% | 0.028 | 0.1 |
| Glutamine 200 mM | 1:100 | 1.06 | 13 |
| Amino acids, nonessential | 1:50 | 0.31 | 3.6 |
| Amino acids, essential | 1:100 | 0.69 | 8.4 |
| Lipid mixture | 0.01% | 0.63 | 7.6 |
| FCS | 0.3% | 0.19 | 2.1 |

The results indicated that all 3 different Primatone batches (Primatone RL was set as 100 %) gave the best results, followed by HySoy and Lactalbumin. Primatone is a cell culture product, a known source for amino acids and small peptides used for many years as a medium supplement for many different cell lines as a serum replacement to increase cell density and cell viability and also to increase yields of recombinant proteins. HySoy (Sheffield Products, Quest International, 10% stock solution in pure water) is a protein hydrolysate from Soja beans. Lactalbumin (LTI, Switzerland) was used as a 10% stock solution in pure water. Yeast extract (Difco, Switzerland) was used as a 10% stock solution in pure water. Glutamine was used as a 200 mM stock solution in pure water and stored at -20 °C. Essential amino acids (LTI, Switzerland) were used as a 100-fold stock solution. Non-essential amino acids (LTI, Switzerland) were used as a 50-fold stock solution. To prepare the lipid mixture, different lipids were dissolved in ethanol and diluted by rigorously mixing with Pluronic F68 according to the description for preparing serum-free insect cell medium (Schlaeger et al, 1995).

### Example 12. Effect of protein hydrolysate on complex stability

1 mg of the compound from Example 2 was dissolved in 0.5 ml acetonitrile mixed for 3-5 minutes at 37 °C and diluted in 0.5 ml sterile, pure water (1 mg/ml) and stored at 4 °C. 90 mg polyethelenimine (PEI), with a molecular weight of 25 kDa, were dissolved in sterile, pure water (0.9 mg/ml), neutralized with HCl, sterile filtered and stored at 4 °C. Primatone RL (sterilized 10% stock solution in pure water) was added to 1.5 ml IS 293 medium (without Primatone) and mixed. After 2-3 minutes, 3 µl of a plasmid solution (1 mg/ml) encoding the luciferase gene was added further and mixed. After 2 minutes incubation at room temperature 2.25 µl of the compound from Example 2 (1mg/ml) was added, mixed, followed by 8.3 µl PEI (0.9 mg/ml). After mixing and incubation for 15-30 minutes at room temperature the mixture was transferred into 15 ml of HEK293-EBNA cells. Cells which were grown in serum-free suspension culture in HL medium (containing 0.3% Primatone RL) were transferred into fresh HL medium (containing 0.3% Primatone RL) then adjusted to 5.5-6 x 10⁶ cells/ml before adding the DNA complexes. The luciferase activity was measured in the soluble cell extract after 20-22 hours incubation time and expressed as relative light units per ml culture.

Table 10 shows the stability of the DNA complexes in the presence and absence of protein hydrolysate in IS293 medium using the compound from Example 2 and PEI.

**Table 10**

| Stability of DNA complexes in IS 293 medium | | | |
|---|---|---|---|
| Incubation time | Temperature | Luciferase activity (RLU x 10⁹/ml) | |
| | | Without protein hydrolysate* | With protein hydrolysate (0.2%)* |
| Omin | 21 °C | 0.18 | 0.76 |
| 5 min | 21 °C | 0.268 | 1.81 |
| 15 min | 21 °C | 1.61 | 6.12 |
| 30 min | 21 °C | 2.00 | 9.41 |
| 75 min | 21 °C | 3.41 | 7.82 |
| 20 hrs | 21 °C | 0.42 | 0.60 |
| 20 hrs** | 4 °C | 1.45 | 5.08 |

| | | | |
|---|---|---|---|
| * Primatone RL | | | |
| ** 15 minutes at 21 °C followed 20 hours at 4 °C. | | | |

The results clearly show that with 0.2% Primatone RL and 30 minutes incubation time, the highest expression levels were achieved in the IS 293 medium.

### Example 13. Effect of antibiotics on expression level using protein hydrolysate

1 mg of the compound from Example 2 was dissolved in 0.5 ml acetonitrile, mixed for 3-5 minutes at 37 °C, diluted in 0.5 ml sterile, pure water (1 mg/ml) and stored at 4 °C. 90 mg polyethelenimine (PEI), with a molecular weight of 25 kDa, were dissolved in sterile, pure water (0.9 mg/ml), neutralized with HCl, sterile filtered and stored at 4 °C. 3 µl of a plasmid solution (1 mg/ml) encoding the luciferase gene was added to 1.5 ml HL medium (containing 0.3% Primatone RL) and mixed. After 2 minutes incubation at room temperature, 2.25 µl of the compound from Example 2 (1mg/ml) was added and mixed, followed by 8.3 µl PEI (0.9 mg/ml). After mixing and incubation for 15-30 minutes at room temperature, the mixture was transferred into 15 ml of HEK293-EBNA cells containing different antibiotics. Cells which were grown in serum-free suspension culture in HL medium (containing 0.3% Primatone RL and different antibiotics) were transferred into fresh HL medium (containing 0.3% Primatone RL and different antibiotics), then adjusted to 5.5-6 x 10⁶ cells/ml before adding the DNA complexes. The luciferase activity was measured in the soluble cell extract after 20-22 hours incubation time and expressed as relative light units per ml culture.

Table 11 shows the expression levels in the presence of different antibiotics in HL medium containing Primatone RL (0.3%).

**Table 11**

| Influences of different antibiotics on transient transfection in HL medium | | |
|---|---|---|
| Antibiotic | Concentration (µg/ml) | Luciferase activity (percent) |
| Control (without) | - | 100 |
| Pen/Strep | 1:100 | 90.3 |
| Gentamycin | 50 | 80 |
| Geneticin (G-418) | 250 | 93 |
| Doxycycline | 2 | 89 |

The results indicated a slight decrease of the expression levels in the presence of different antibiotics. Penicillin/Streptomycin (110-fold concentrated) and Gentamycin were from LTI, and Geneticin and Doxycyclin were from Sigma.

### Example 14. DNA complex formation using different transfection reagents

Many different culture media were tested in the presence and in the absence of protein hydrolysate for the formation of highly active transfection complexes. The results clearly showed that a high variation in expression levels was observed in different media, whereby the addition of protein hydrolysates led in several cases to a significantly increased expression activity of 2- to 100-fold. The positive effect of protein hydrolysate on the DNA complex formation was also observed when other transfection reagents were tested. The enhancement of the transfection efficiency when the DNA complex formation was performed in the presence of protein hydrolysate is not yet understood.

1 mg of the compound from Example 2 was dissolved in 0.5 ml acetonitrile, mixed for 3-5 minutes at 37 °C, diluted in 0.5 ml sterile, pure water (1 mg/ml) and stored at 4 °C. 90 mg polyethelenimine (PEI), with a molecular weight of 25 kDa, were dissolved in sterile, pure water (0.9 mg/ml), neutralized with HCl, sterile filtered and stored at 4 °C.

1 µl of a plasmid solution (1 mg/ml) encoding the luciferase gene was added to 0.5 ml different medium and mixed. After 2 minutes incubation at room temperature, 0.75 µl of the compound from Example 2 (1mg/ml) was added and mixed, followed by different amounts of different transfection reagents. After mixing and incubation for 15-30 minutes at room temperature, the mixture was transferred into 1.5 ml of HEK293-EBNA cells in Bellco incubation tubes. Cells which were grown in serum-free suspension culture in HL medium (containing 0.3% Primatone RL) were transferred into fresh HL medium (containing 0.3% Primatone RL), then adjusted to 5.5-6 x 10⁶ cells/ml before adding the DNA complexes. The tubes were rotated in a roller drum during incubation. The luciferase activity was measured in the soluble cell extract after 20-22 hours incubation time and expressed as relative light units per ml culture.

**Table 12**

| Sample | Medium | Transfection Reagent | Without protein hydrolysate RLU/ml [x 10⁹] | With protein hydrolysate RLU/ml [x 10⁹] |
|---|---|---|---|---|
| Blank | | | 0.00178 | 0.00178 |
| Control sample | | | 18.74 | 18.74 |
| 1 | D/F12* | LipofectAMINE | 0.50 | 0.97 |
| 2 | D/F12 | LipofectAMINE 2000 | 6.78 | 9.04 |
| 3 | D/F12 | DMRIE-C | 0.94 | 2.10 |
| 4 | D/F12 | Fugene 6 | 9.22 | 9.16 |
| 5 | D/F12 | PolyFect (Qiagen) | 1.97 | 1.79 |
| 6 | D/F12 | Transfectam | 2.80 | 2.60 |
| 7 | D/F12 | PEI (25 kDa) | 0.056 | 3.61 |
| 8 | D/F12 | Cpd. from Ex. 2/PEI | 0.045 | 12.36 |
| 9 | IS 293** | Cpd. from Ex. 2/PEI | 1.84 | 12.32 |
| 10 | IS 293 | Fugene 6 | 9.83 | 9.81 |
| 11 | MEM α (LTI) | Lipo 2000 | 4.88 | 6.29 |
| 12 | Optimem (LTI) | Lipo 2000 | 5.15 | 9.11 |
| 13 | IS 293 | Lipo 2000 | 4.62 | 5.31 |
| 14 | HL (8) control | Cpd. from Ex. 2/PEI | 5.05 | 12.00 |

| | | | | |
|---|---|---|---|---|
| * DMEM HAM/F12 (LTI) | | | | |
| ** IS 293 (Irvine Science) | | | | |

## Claims

1. A conjugate of the formula 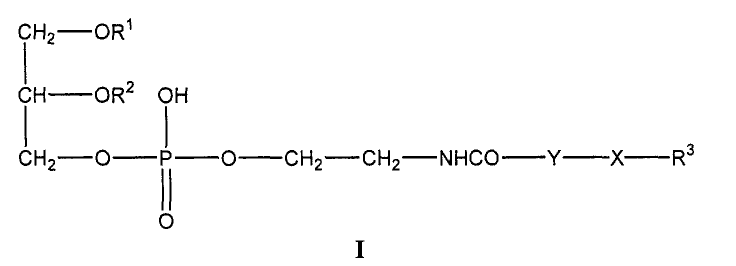 wherein R¹ and R² are selected from the group consisting of hydrocarbyl moieties of straight chain and branched-chain, saturated and unsaturated aliphatic carboxylic acids and phospholipid moieties, R³ is a basic, membrane-disturbing peptide with a reversed amide backbone, Y is C₂₋₁₀ alkylene, and X is selected from the group consisting of -C(O)-NH-, -S-S-, and salts thereof.

2. The conjugate of claim 1, wherein R¹ and R² are acyl moieties of a C₁₂₋₂₀ carboxylic acid.

3. The conjugate of claim 1 wherein R¹ and R² are selected from the group consisting of lauroyl, palmitoyl, stearoyl and oleoyl.

4. The conjugate of claim 1 wherein X is -S-S-.

5. The conjugate of claim 1 wherein Y is selected from the group consisting of ethylene, propylene and decamethylene.

6. The conjugate of claim 1 wherein R³ is Gln-Gln-Arg-Lys-Arg-Lys-Ile-Trp-Ser-Ile-Leu-Ala-Pro-Leu-Gly-Thr-Thr-Leu-Val-Lys-Leu-Val-Ala-Gly-Ile-NH-CH[CONH₂]-(CH₂)- comprising at least 50% D-amino acids.

7. The conjugate of claim 1 wherein R³ is D-Gln-D-Gln-D-Arg-D-Lys-D-Arg-D-Lys-D-Ile-D-Trp-D-Ser-D-Ile-D-Leu-D-Ala-D-Pro-D-Leu-D-Gly-D-Thr-D-Thr-D-Leu-D-Val-D-Lys-D-Leu-D-Val-D-Ala-D-Gly-D-Ile-NH-CH[CONH₂]-CH-(CH₂)-.

8. The compound

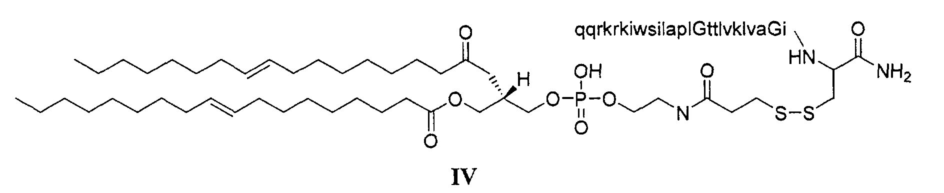

9. The peptide Gln-Gln-Arg-Lys-Arg-Lys-Ile-Trp-Ser-Ile-Leu-Ala-Pro-Leu-Gly-Thr-Thr-Leu-Val-Lys-Leu-Val-Ala-Gly-Ile-Cys-NH₂ and derivatives, salts and solvates thereof comprising at least 50% D-amino acids.

10. The peptide D-Gln-D-Gln-D-Arg-D-Lys-D-Arg-D-Lys-D-Ile-D-Trp-D-Ser-D-Ile-D-Leu-D-Ala-D-Pro-D-Leu-D-Gly-D-Thr-D-Thr-D-Leu-D-Val-D-Lys-D-Leu-D-Val-D-Ala-D-Gly-D-Ile-D-Cys-NH₂ and derivatives, salts and solvates thereof.

11. A reagent composition comprising a conjugate of the formula 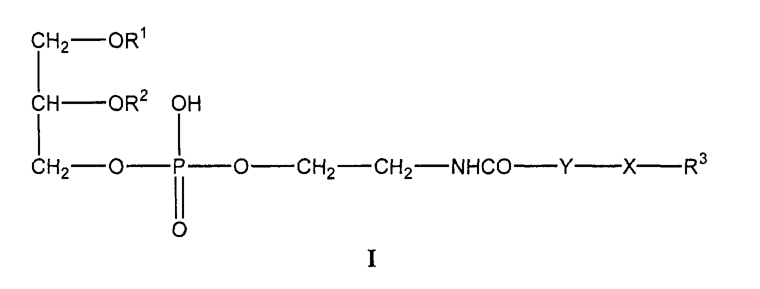 wherein R¹ and R² are selected from the group consisting of hydrocarbyl moieties of straight chain and branched-chain, saturated and unsaturated aliphatic carboxylic acids and phospholipid moieties, R³ is a basic, membrane-disturbing peptide with a reversed amide backbone, Y is C₂₋₁₀ alkylene, and X is selected from the group consisting of -C(O)-NH-, -S-S-, and salts thereof, and a transfection-competent molecule selected from the group consisting of helper lipids, short chained phospholipids and cationic lipids.

12. The composition of claim 11 further comprising an anionic macromolecule selected from the group consisting of polynucleotides and proteins.

13. The composition of claim 11 further comprising a polycationic polymer.

14. The composition of claim 13 wherein said polycationic polymer is polyethyleneimine.

15. The composition of claim 11 further comprising a protein hydrolysate.

16. A reagent composition comprising a compound according to claim 8 and polyethyleneimine.

17. The composition of claim 16 further comprising a protein hydrolysate.

18. A method for introducing a biologically active molecule into a eukaryotic cell comprising the steps of:
(a) providing a medium for DNA complex formation,
(b) in said medium, contacting said cell with said biologically active molecule in the presence of a conjugate according to claim 1, and further in the presence of a transfection-competent molecule selected from the group consisting of helper lipids, short chained phospholipids and cationic lipids.

19. The method of claim 18 wherein said contacting step is performed further in the presence of a protein hydrolysate.

20. The method of claim 19 wherein said protein hydrolysate is a tryptic meat digest.

21. The method of claim 18 wherein said biologically active molecule is an anionic macromolecule selected from the group consisting of polynucleotides and proteins.

22. A method for introducing a biologically active molecule into a eukaryotic cell comprising the steps of:
(a) providing a medium for DNA complex formation,
(b) in said medium, contacting said cell with said biologically active molecule in the presence of a compound according to claim 8, and further in the presence of a transfection-competent molecule selected from the group consisting of helper lipids, short chained phospholipids and cationic lipids.

23. The method of claim 22 wherein said contacting step is performed further in the presence of a protein hydrolysate.

24. The method of claim 23 wherein said protein hydrolysate is a tryptic meat digest.

25. The method of claim 22 wherein said biologically active molecule is an anionic macromolecule selected from the group consisting of polynucleotides and proteins.
